# EUROPEAN PATENT SPECIFICATION

(11) **EP 2 563 407 B1**
(45) Date of publication and mention of the grant of the patent: **19.07.2017**
(21) Application number: 11721638.2
(22) Date of filing: 28.04.2011
(51) Int. Cl.: A61K 49/00

(54) **DIAGNOSTIC REAGENTS**
DIAGNOSTISCHE REAGENZIEN
RÉACTIFS DE DIAGNOSTIC

(30) Priority: 28.04.2010 GB 201007075
(43) Date of publication of application: 06.03.2013
(73) Proprietor: The Secretary of State for Environment, Food & Rural Affairs, Addlestone Surrey KT15 3NB (GB)
(72) Inventor: VORDERMEIER, Hans, Addlestone Surrey KT15 3NB (GB); WHELAN, Adam, Addlestone Surrey KT15 3NB (GB)
(74) Representative: Turner, Rhiannon Rosalind
(86) International application number: PCT/GB2011/050843
(87) International publication number: WO 2011/135369

(56) References cited:
- WO-A1-2008/135067
- WO-A1-2009/060184
- US-A1- 2006 024 332
- SIDDERS BEN ET AL: "Screening of highly expressed mycobacterial genes identifies Rv3615c as a useful differential diagnostic antigen for the Mycobacterium tuberculosis complex.", INFECTION AND IMMUNITY SEP 2008 LNKD- PUBMED:18519559, vol. 76, no. 9, September 2008 (2008-09), pages 3932-3939, XP002651138, ISSN: 1098-5522
- MACGURN JASON A ET AL: "A non-RD1 gene cluster is required for Snm secretion in Mycobacterium tuberculosis.", MOLECULAR MICROBIOLOGY SEP 2005 LNKD- PUBMED:16135231, vol. 57, no. 6, September 2005 (2005-09), pages 1653-1663, XP002651139, ISSN: 0950-382X

## Description

### Field of invention

The present invention relates to reagents for use in a skin test for detection of mycobacterium infections, particularly *Mycobacterium tuberculosis* and *M. bovis,* in mammals such as cattle.

### Background

*M. tuberculosis* and *M. bovis* are important pathogens of man and animals. *M. tuberculosis* is thought to infect up to a third of the world's human population, remaining undetected during a latent phase of infection and reactivating to cause 10 million cases of tuberculosis and other diseases per year, resulting in 2 million deaths (Corbett et al. (2003) Arch. Intern. Med. vol. 163 pp 1009-1021). *M. bovis,* which has more than 99.9% sequence identity with *M. tuberculosis,* is the causative agent of bovine tuberculosis (BTB) and also causes disease in human. BTB represents a significant economic burden to the agricultural industries of various countries including the United Kingdom (Krebs (1997) "Bovine Tuberculosis in Cattle & Badgers" HMSO, London, United Kingdom).

The primary diagnostic test used in the control and surveillance of bovine TB is the tuberculin skin-test, a test that has remained in the forefront of TB diagnosis in both man and cattle for over 100 years. The development of the test arose following the preparation of the first 'tuberculin' by Robert Koch in 1890. Whilst Koch's tuberculin failed to live up to its initial claims of having curative properties, its diagnostic potential was quickly realised. The most common formats of the test used in cattle are the caudal fold test (CFT), the single intradermal cervical tuberculin test (SIT) and the single intradermal comparative cervical tuberculin (SICCT) test (Monaghan et al. (1994) Vet. Microbiol. vol. 40 pp 111-24). These test formats use a purified protein derivative (PPD) tuberculin prepared from a culture of *M. bovis* (PPD-B) as the primary diagnostic antigen. Additionally, the SICCT test includes the use of a *M. avium* derived PPD (PPD-A) to provide a measure of environmental sensitisation. It is the most specific of the tests (Plum (1931) Cornell Vet. vol. 21 pp 68-76; Stenius (1938) Veterinary Record vol. 50 pp 633-7) and is, therefore, the adopted test format in the UK.

In addition to skin tests, blood-based diagnostic assays that measure antigen-induced lymphokine production such as the interferon gamma (IFN-γ) test are also under consideration. The cytokine IFN-γ appears to be critical in the development of immunity to *M. tuberculosis.* For example, mice with a disrupted IFN-γ gene and also humans with a mutated IFN-γ receptor are highly susceptible to mycobacterial infections. However, specificity constraints are associated with the use of PPD in such assays. These arise due to the crude mixture of *M. bovis* proteins that PPD contains, many of which are cross-reactive with the BCG vaccine strain and environmental mycobacterial species such as *M. avium* and *M. intracellulare.*

Bovine TB is a significant and ongoing problem in the UK (http://www.defra.gov.uk/food-farm/animals/diseases/tb/, accessed 18 April 2011). Cattle vaccination has been identified as one of the most promising long term UK control strategies (Krebs (1997) "Bovine Tuberculosis in Cattle & Badgers" HMSO) and the development of an efficacious vaccine continues to be a research priority. Currently, promising vaccines against bovine TB are based on heterologous prime-boost combinations that include the live attenuated *M. bovis* vaccine strain Bacille Calmette-Guerin (BCG) as one of their components (Hogarth et al. (2006) J. Pharm. Pharmacol. vol. 58 pp 749-57). However, as in humans, vaccination of cattle with BCG compromises the specificity of the tuberculin skin-test since PPD contains cross reactive antigens shared by both pathogenic and vaccine strains (Berggren (1981) Br. Vet. J. vol. 137 pp 88-94; Buddle et al. (1999) Clin. Diagn. Lab. Immunol. vol. 6 pp 1-5; Waddington & Ellwood (1972) Br. Vet. J. vol. 128 pp 541-52). Therefore, the development of diagnostic tests that can differentiate vaccinated from infected animals, so-called DIVA tests, are an essential pre-requisite to allow the inclusion of BCG-based vaccination as part of bovine TB control strategies.

Previous studies have demonstrated that diagnostic reagents which distinguish between vaccinated and infected cattle can be developed using specific, defined antigens that are present in virulent *M. bovis* but absent from the BCG. Genetic analysis of BCG has revealed that several large genomic regions have been deleted during attenuation and subsequent prolonged propagation in culture. These regions have been characterised and antigens from one of these regions, RD1, have been studied extensively in several species including humans and cattle. For example, it has been demonstrated that protein or polypeptide cocktails composed of two RD1 region antigens, ESAT-6 and CFP-10, can be used to distinguish between *M. bovis* infected and BCG-vaccinated cattle.

The practical application of such DIVA reagents have so far been largely realised through their use in blood-based interferon-γ (IFN-γ) release assays (IGRAs). For example, WO2009/060184 and Sidders et al. (2008; Infect. Immun. vol. 76 p 3932-3939) disclosed several polypeptides including epitopes from Rv3615c which were found to be useful to detect *M. bovis* infection, using such an assay. This polypeptide has also been confirmed as useful to detect *M. tuberculosis* infection in humans (Millington et al. (2011) Proc. Natl. Acad. Sci. USA vol. 108 pp 5730-5735). Rv3615c is referred to herein using the *M. tuberculosis* genome annotation (http://genolist.pasteur.fr/TubercuList/, accessed 18 April 2011). In the *M. bovis* genome, it is annotated as Mb3645c (http://genolist.pasteur.frBoviList/, accessed 18 April 2011). MacGurn et al. (Mol. Microbiol. (2005) vol. 57 p.1653-63) discusses the impact of a transposon insertion in the Rv3615c gene on Snm secretion.

Given the high level of familiarity and wide-spread application of the tuberculin skin-test by veterinarians and clinicians, a DIVA skin-test format would provide a valuable additional test platform. This might especially be the case where the logistics of access to laboratory-based resources is problematic. It is also notable that, in recent years, there has also been renewed interest in a skin-test based DIVA test for human TB with several reports demonstrating the skin-test potential of ESAT-6 (Aggerbeck & Madsen (2006) Tuberculosis (Edinb.) vol. 86 pp 363-73; Arend et al. (2000) J. Infect. Dis. vol. 181 pp 1850-4; Wu et al. (2008) Clin. Exp. Immunol. vol. 152 pp 81-7). However, Whelan et al. (Infect. Immun. (2003) vol. 71 pp 6420-6425) found that ESAT-6 was not effective on its own as a skin test reagent in cattle assessed using measurement of skin induration at the injection site. The addition of a synthetic bacterial lipopeptide adjuvant was required before ESAT-6 proved useful in cattle. It is preferable to avoid the use of adjuvants in a skin test reagent, since repeat skin test injections (as is required to monitor the health of, for example, a herd of dairy cattle) may lead to the sensitisation of non-tuberculosis infected animals, so that the skin test would cease to be useful to differentiate between infected animals and uninfected but vaccinated animals.

The present invention accordingly addresses the problem of providing discriminatory diagnostic reagents for the detection of mycobacterial infections using a DIVA skin-test format.

### Summary of Invention

According to a first aspect of the invention, there is provided a skin test diagnostic reagent in the form of a sterile injectable preparation comprising the CFP-10 epitope polypeptides consisting of amino acid sequences SEQ ID NOs: 1-10; the ESAT-6 epitope polypeptides consisting of amino acid sequences SEQ ID NOs: 11-21; and the Rv3615c epitope polypeptides consisting of amino acid sequences SEQ ID NOs: 23-34. The diagnostic reagent is capable of eliciting a positive result when administered in a skin test to an animal infected with *Mycobacterium bovis* or *Mycobacterium tuberculosis.* In one embodiment, the reagent does not comprise an adjuvant, i.e., a reagent that assists in propagating an immune response to enhance the effect of the diagnostic reagent but which does not itself induce an immune response. An example is a bacterial lipopeptide and the skilled person is readily able to determine the identity of a suitable adjuvant in a given context.

This invention arises from the inventors' discovery that this particular combination of polypeptides provides unexpectedly good results when used in a skin test for bovine tuberculosis. CFP-10 has not previously been assessed for use in a skin test. Furthermore, the inventors have found that ESAT-6 is only useful in a skin test in cattle when used in conjunction with an adjuvant. Advantageously, the reagent according to the claims enables the use of a skin test capable of distinguishing between *M. bovis* and/or *M. tuberculosis* infected animals and animals which have been vaccinated against infection by *M. bovis* and/or *M. tuberculosis,* for example, with a BCG vaccine.

The animal may be a mammal such as a cow, a badger or a human being.

The term "epitope polypeptide", as used throughout this specification, indicates a polypeptide which includes one or more (or all) epitopes of the relevant protein. The term "epitope" refers to the amino acids (typically a group of around 5 or more amino acids) within a polypeptide sequence which are essential in the generation of an immune response and which can, therefore, be used in a diagnostic test. The immune response may be an antibody mediated immune response, but may also be a non-antibody mediated immune response, for example, an immune response which can be detected by means of a cell-mediated immunity (CMI) assay. Therefore, the epitope may be one which is recognisable by a T cell, for example by binding of a T cell receptor to the epitope.

The skin test referred to herein may be any of a CFT, SIT or SICCT test, as described in the Office International des Epizooties (OIE) Manual of Diagnostic Tests and Vaccines for Terrestrial Animals (ISBN-10:92-9044-718-4; http://www.oie.int/eng /normes/mmanual/a_summry.htm, accessed 18 April 2011). The manual provides information, definitions and guidelines on positive test criteria. Therefore, when the diagnostic reagent described herein elicits a positive result when administered in a skin test such as one of those mentioned above, this is determined, for example, by detection of an increased thickness and/or induration of skin at the site at which the diagnostic reagent has been injected, using callipers, for example. The skin thickness may ideally be determined, for example, prior to injection (to provide a starting thickness for comparison after injection) and at one or more of, for example, about 24, 36, 48, 72, 96 or about 120 hours after injection of the diagnostic reagent. Determining skin thickness at about 72 hours after injection is typical. Thickness may be determined at any time period after injection, provided that, when results from different tests are compared, they are compared after substantially the same time period after injection (e.g., between 1 and 10 hours before or after one of the time points mentioned above such as the 72 hour time point, for example, between 3 and 7 hours before or after or about 5 hours before or after).

The diagnostic reagent may further comprise one or more of the three polypeptides having amino acid sequences SEQ ID NOs:35, 36 and 38, i.e., full length CFP-10, ESAT-6 and Rv3615c, respectively. In one embodiment, the diagnostic reagent comprises all of SEQ ID NOs:35, 36 and 38.

The diagnostic reagent comprises the amino acid sequences SEQ ID NOs:1-10. These sequences are polypeptides which are overlapping fragments of CFP-10 which are CFP-10 epitope polypeptides, i.e., each of them contains at least one CFP-10 epitope.

The diagnostic reagent comprises the amino acid sequences SEQ ID NOs:11-21. These sequences are polypeptides which are overlapping fragments of ESAT-6 which are ESAT-6 epitope polypeptides, i.e., each of them contains at least one ESAT-6 epitope.

The diagnostic reagent comprises the amino acid sequences SEQ ID NOs:23-34. These sequences are polypeptides which are overlapping fragments of Rv3615c which are Rv3615c epitope polypeptides, i.e., each of them contains at least one Rv3615c epitope. The diagnostic reagent may further comprise at least one of the polypeptides having amino acid sequences SEQ ID NOs:22 or 37.

The diagnostic reagent may further comprise at least one MPB83 epitope polypeptide, for example a polypeptide having amino acid sequence SEQ ID NO:37. This sequence is the amino acid sequence of MPB83 (identified in WO97/08322). The inventors surprisingly found that including this protein elicited more accurate and sensitive results when combined with CFP-10 and ESAT-6 proteins, even though MPB83 protein alone has poor IFN-γ inducing capacity, in comparison to CFP-10 and ESAT-6. Alternatively or additionally, the diagnostic reagent may comprise at least one polypeptide having amino acid sequence SEQ ID NO:22. This sequence is a MPB83 epitope polypeptide, i.e., it contains at least one MPB83 epitope.

The diagnostic reagent may further comprise at least one MPB70 epitope polypeptide, for example a polypeptide having amino acid sequence SEQ ID NO:39. This sequence is the amino acid sequence of MPB70.

The polypeptides may be included in the diagnostic reagent in individual form, or in the form of one or more fusion proteins. For example, the diagnostic reagent may comprise a fusion protein comprising SEQ ID NOs: 35, 36 and 38.

The diagnostic reagent may be for use in a method of detecting *Mycobacterium bovis* or *Mycobacterium tuberculosis* infection in an animal using a skin test.

The sterile injectable preparation may be an aqueous or an oleaginous suspension, or a suspension in a non-toxic parenterally-acceptable diluent or solvent. The aqueous suspension may be prepared in, for example, mannitol, water, Ringer's solution or isotonic sodium chloride solution. Alternatively, it may be prepared in phosphate buffered saline solution. The oleaginous suspension may be prepared in a synthetic monoglyceride, a synthetic diglyceride, a fatty acid or a natural pharmaceutically-acceptable oil. The fatty acid may be an oleic acid or an oleic acid glyceride derivative. The natural pharmaceutically-acceptable oil may be an olive oil, a castor oil, or a polyoxyethylated olive oil or castor oil. The oleaginous suspension may contain a long-chain alcohol diluent or dispersant, for example, Ph. Helv.

In an embodiment, the diagnostic reagent is able (and, therefore, allows a user) to differentiate between an *M. bovis*- and/or *M. tuberculosis*-infected animal and an animal vaccinated against *M. bovis* or *M. tuberculosis* (for example, an animal vaccinated with the live attenuated vaccine BCG).

"Using" and "use" of polypeptides and diagnostic reagents in the skin test typically involves intradermal injection of the polypeptide(s) and/or diagnostic reagent into the animal. A skin test may be conducted as described in OIE Manual of Diagnostic Tests and Vaccines for Terrestrial Animals, as mentioned above.

According to a second aspect of the invention, there is provided a diagnostic kit comprising a diagnostic reagent according to the first aspect of the invention. The diagnostic reagent may be in liquid form, as outlined above, or may be in solid (for example, lyophilised) form. It may be included in the kit in the form of at least one aliquot of 0.05-0.15ml containing 1-15µg of each polypeptide contained in the diagnostic reagent. For example, the kit may comprise aliquots of about 0.05ml, about 0.06ml, about 0.07ml, about 0.08ml, about 0.09ml, about 0.1ml, about 0.11ml, about 0.12ml, about 0.13ml, about 0.14ml or about 0.15ml, containing 1-15µg, for example, 3-12µg or 5-10µg of each epitope polypeptide, for example, about 5µg, about 6µg, about 7µg, about 8µg, about 9µg or about 10µg of each epitope polypeptide. Each aliquot may be contained in a disposable injection device. The kit may further comprise at least one sample of PPD. The diagnostic reagent may be able to detect a *M. bovis* or *M. tuberculosis* infection in a mammal and may be able to differentiate between an *M. bovis-* or *M. tuberculosis*-infected mammal and a mammal vaccinated against infection by *M. bovis* or *M. tuberculosis.*

According to a third aspect of the invention there is provided a diagnostic reagent according to a first aspect of the invention for use in a method for diagnosing infection of an animal by *Mycobacterium bovis* or *Mycobacterium tuberculosis,* the method comprising the steps of conducting a skin test on the animal using the diagnostic reagent and correlating a positive skin test result with infection of the animal by *Mycobacterium bovis* or *Mycobacterium tuberculosis,* hence diagnosing the animal as being infected with *Mycobacterium bovis* or *Mycobacterium tuberculosis.*

Throughout the description and claims of this specification, the words "comprise" and "contain" and variations of the words, for example "comprising" and "comprises", mean "including but not limited to" and do not exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Any features of each aspect of the invention may be as described in connection with any of the other aspects.

Other features of the present invention will become apparent from the following examples. Generally speaking, the invention extends to any novel one, or any novel combination, of the features disclosed in this specification (including the accompanying claims and drawings). Thus, features, integers, characteristics, compounds or chemical moieties described in conjunction with a particular aspect, embodiment or example of the invention are to be understood to be applicable to any other aspect, embodiment or example described herein, unless incompatible therewith.

Moreover, unless stated otherwise, any feature disclosed herein may be replaced by an alternative feature serving the same or a similar purpose.

### Brief Description of Figures

Particular non-limiting examples of the present invention will now be described with reference to the following Figures, in which:
Figure 1 shows protein-cocktail induced skin-test responses in cattle, with A showing comparative SICCT PPD responses [(PPD-B)-(PPD-A)] and B showing responses induced by a cocktail of 10µg each of the proteins ESAT-6, CFP-10, MPB70;
Figure 2 shows the *in vitro* capacity of antigens to induce IFN-γ in blood from cattle naturally exposed to *M. bovis* (n=37), determined for the proteins ESAT-6, CFP-10, MPB70 and MPB83 either individually (5µg/ml) or as a combined protein cocktail (5µg/ml per constituent);
Figure 3 shows skin-test dose titration protein-cocktail comprising ESAT-6, CFP-10, MPB70 and MPB83 proteins in cattle that were naturally exposed to *M. bovis* (n=19);
Figure 4 shows skin-test specificity of the protein cocktail compromising ESAT-6, CFP-10, MPB70 and MPB83 proteins (10µg for each component) and for the SICCT-test [(PPD-B)-(PPD-A)] in naive TB-free (n=19) and BCG vaccinated (n=20) cattle;
Figure 5 shows skin-test responses induced by the SICCT-test [(PPD-B)-(PPD-A)] and a selection of protein and protein fragment polypeptide based antigen combinations, determined in cattle naturally exposed to *M. bovis* (n=13);
Figure 6 shows skin-test responses induced by ESAT-6, CFP-10, MPB83 and Rv3615c proteins, determined either individually (10µg) or in combination (10µg per protein) in cattle naturally exposed to *M. bovis* (n=12);
Figure 7 shows the skin-test performance of an ESAT6/CFP10 based diagnostic protein fragment polypeptide cocktail that additionally includes 11 Rv3615c protein fragment polypeptides, measured in SICCT-test positive (reactor) cattle (n=15) ; and
Figure 8 shows the skin-test performance of an ESAT6/CFP10/MPB83/Rv3615c protein cocktail, an ESAT6/CFP10/Rv3615c protein cocktail, an ESAT6/CFP10 protein fragment polypeptide cocktail and an ESAT6/CFP10/Rv3615c protein fragment polypeptide cocktail, measured in SICCT-test positive (reactor) cattle (n=15).

### Examples

### Materials and Methods

### Animals

To investigate the skin-test performance of defined antigens in cattle naturally exposed to *M. bovis,* SICCT reactor cattle were recruited from UK farms with a confirmed history of bovine TB (convenience sampling dependent on availability). Animals were identified during routine surveillance operations where their SICCT response [(PPD-B)-(PPD-A)] >2mm and housed in secure segregated bio-containment facilities at the Veterinary Laboratories Agency (VLA). A total of 62 reactors were used in the study which included 21 male and 41 female cattle of 7 different breeds, including dairy and beef stock. Age at time of study skin-test investigations ranged from 9 months to 11 years, with a median age of 21 months. All reactors underwent detailed post mortem examination to assess for presence of *M. bovis* infection, in accordance with previously described procedures (Vordermeier et al. (2002) Infect. Immun. vol. 70 pp 3026-32). Visible lesions consistent with bovine TB and/or culture isolation of *M. bovis* were confirmed in 55 of these reactors.

For the specificity studies, Holstein-Friesian castrated male cattle (n=54) were sourced from UK TB-free farms and housed at VLA in separate accommodation from the reactors. A cohort of these calves (n=20) were neo-natally vaccinated with BCG to allow DIVA evaluation of reagents. A single dose of 1 x 10⁶ CFU of BCG Danish (Staten Serum Institute, Sweden) was administered subcutaneously to each of these calves at 4-6 weeks of age. Where a second skin-test was performed in some cattle during the study, the second skin-test was performed no earlier than 62 days later. All cattle experiments were cleared by local ethical review and animal procedures under a licence granted by the British Home Office.

### Antigens

Bovine and avian PPDs were supplied by the VLA Tuberculin Production Unit. Histidine tagged recombinant proteins were expressed in *Escherichia coli* and purified by nickel affinity chromatography.

MPB70, MPB83, ESAT-6 and CFP-10 were supplied by Lionex Diagnostics and Therapeutics GmbH (Germany) and Rv3615c was produced at the Agri-Food and Biosciences Institute (Belfast, Northern Ireland). The Rv3615c synthetic protein fragment polypeptides (SEQ ID NOs:23-34) were synthesised by solid phase F-moc chemistry and supplied at a purity of >90% (Pepceuticals Ltd, UK). The identity of each polypeptide was confirmed by mass spectrometry. For ESAT-6 and CFP-10, 16mer protein fragment polypeptides with 8 amino acid overlaps of the complete protein sequences were used (21 polypeptides in total, having SEQ ID NOs:1-10 for CFP10 polypeptides and SEQ ID NOs:11-21 for ESAT-6 polypeptides).

For MPB83, a single 20mer protein fragment polypeptide (SEQ ID NO:22) that had previously been shown to encode a bovine T-cell epitope (p195-214) was used (Vordermeier et al. (1999) Clin. Diagn. Lab. Immunol. vol. 6 pp 675-82). Similarly, for Rv3615c, in initial experiments three bovine T-cell epitope-encoding 20mer protein fragment polypeptides were used (p65-84 (SEQ ID NO:31), p73-92 (SEQ ID NO:32) and p84-103 (SEQ ID NO:34)) (Sidders et al. (2008) Infect. Immun. vol. 76 pp 3932-9). In subsequent experiments, a cocktail of SEQ ID NOs:23-33 was used.

All proteins and polypeptides and cocktails were prepared in phosphate buffered saline (PBS), pH7.4, for use in the following experiments.

### Skin test procedures

The disclosing SICCT field-test was performed and interpreted according to OIE Manual of Diagnostic Tests and Vaccines for Terrestrial Animals. For the evaluation of defined antigen combinations at VLA, up to 8 intradermal injection sites were used on each animal, 4 on each side of the neck. PPD-B and PPD-A (2500 IU) were always included at each test and all antigens were administered in a 0.1ml volume. Defined antigens were tested at a concentration 10µg whether used individually or as a component of a cocktail unless otherwise stated. Skin-induration at administration sites was measured using callipers by the same operator for all experiments. Measurements were recorded prior to and at 72 hours post skin-test and, additionally, at 96 and 120 hours for some experiments. Results are expressed as the difference in skin thickness (mm) between the pre and post skin-test readings.

### IFN-γ whole blood ELISA

Heparinised whole blood cultures were stimulated with either PPD-A (10µg/ml), PPD-B (10µg/ml), recombinant protein antigens (5µg/ml), protein cocktail (5µg/ml per constituent), staphylococcal enterotoxin B (SEB) (1µg/ml Sigma-Aldrich, UK) or no-antigen control. Cultures were set up within 8 hours of blood collection and cultured at 37°C/5% CO₂. Antigen stimulated IFN-γ was measured in 24 hour whole blood culture supernatants using the commercially available BOVIGAM^{®} ELISA (Prionics AG, Switzerland). Results are expressed as the background-corrected optical density measured at 450nm (OD₄₅₀ₙₘ).

### Statistical Analysis

Differences in response magnitude between different antigen combinations were compared by repeated measures ANOVA, with application of Bonferroni's Multiple Comparison post-analysis test. Differences between antigen responder frequencies were analysed using the Fisher Exact Test. These statistical analyses and Receiver Operator Curve (ROC) analysis were performed using the Prism 5 software program (Graphpad Inc, USA).

### Results

### A defined protein cocktail of MPB70, MPB83, ESAT-6 and CFP-10 induces skin-test responses in naturally infected cattle

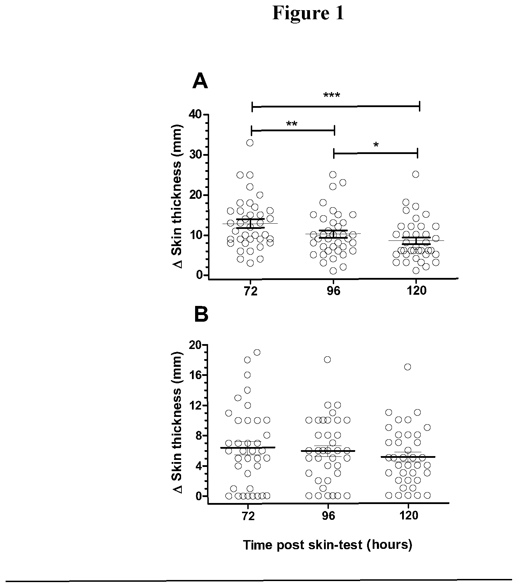

To assess the potential of defined antigens as diagnostic skin-test reagents, the inventors initially formulated a cocktail comprising the purified recombinant *M*. *bovis*/*M. tuberculosis* protein antigens ESAT-6, CFP-10, MPB70 and MPB83. MPB70 and MPB83 were included in the cocktail since they had previous demonstrated DIVA skin-test potential in guinea model of *M. bovis* infection (Vordermeier et al. (1999) Clin. Diagn. Lab. Immunol. vol. 6 pp 675-82). In 37 SICCT positive cattle, the protein cocktail induce a response in 29/37 (78%) of these cattle at 72 hours post-test (Figure 1). The increase in skin induration for each animal is represented by an open circle, the horizontal line provides the mean (±SEM) with results expressed as the difference in skin thickness (mm) between the pre and post skin-test readings. Statistical difference between responses was determined using by ANOVA (* p<0.05, ** p<0.01, *** p<0.001).

Skin reactions were additionally measured at 96 and 120 hours since it has previously been reported that the development of bovine skin-test responses to ESAT-6 continued to increase after 72 hours (Pollock et al. (2003) J. Clin. Microbiol. vol. 41 pp 1856-60). In comparison with the 72 hour reactions, the SICCT responses were significantly reduced at 96 and 120 hours post-test (Figure 1). The magnitude of protein cocktail-induced reactions was comparable at each time point.

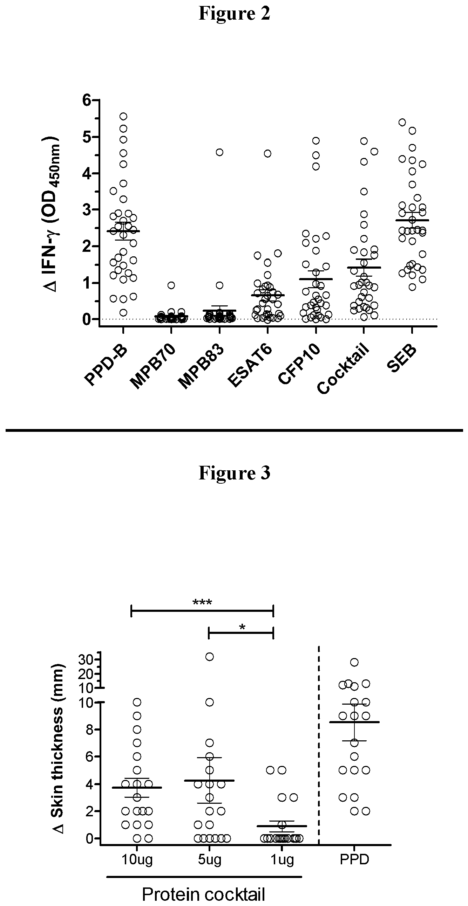

Measurement of antigen induced IFN-γ in whole blood cultures set up on the day of the skin-test demonstrated that ESAT-6 and CFP-10 were the most strongly recognised constituent antigens in the protein cocktail *in vitro* (Figure 2). The *in vitro* capacity of antigens to induce IFN-γ in blood from cattle naturally exposed to *M. bovis* (n=37) was determined for ESAT-6, CFP-10, MPB70 and MPB83 either individually (5µg/ml) or as a combined protein cocktail (5µg/ml per constituent). Responses induced by PPD-B (10µg/ml), SEB (1µg/ml) or no-antigen control were also determined. The antigen induced IFN-γ response for each animal is represented by an open circle, the horizontal line provides the mean (± SEM) with results expressed as the background-corrected optical density measured at 450nm (OD₄₅₀ₙₘ).

The protein cocktail was next tested at a titrated dose concentration of 10, 5 and 1µg for each cocktail component. The respective responses in a cohort of 19 of the previously tested reactors are shown in Figure 3. A skin-test dose titration was determined for the protein cocktail compromising ESAT-6, CFP-10, MPB70 and MPB83 in cattle that were naturally exposed to *M. bovis* (n=19). The protein cocktail was administered at a concentration of 10, 5 and 1 µg for each antigen component. Protein cocktail and SICCT [(PPD-B)-(PPD-A)] responses measured at 72 hours for each animal are represented by an open circle, the horizontal line provides the mean (±SEM) with results expressed as the difference in skin thickness (mm) between the pre and post skin-test readings. Statistical difference between responses induced by the protein cocktails was determined using by ANOVA (* p<0.05, *** p<0.001). Skin-test responses were comparable between the 10 and 5µg dose but significantly reduced at the 1µg dose demonstrating a dose response. For all further experiments, a skin-test concentration of 1µg per defined antigen component was used.

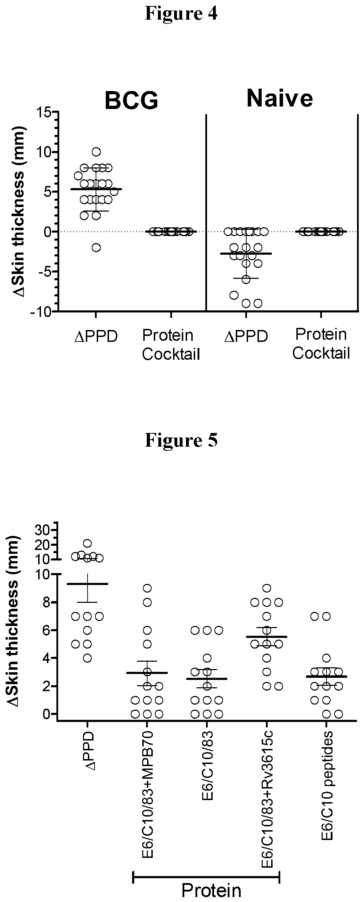

The specificity of this protein cocktail was first determined in naive cattle (n=20). The cocktail induced no measureable response in any of these animals when reactions were read at 72 hours (Figure 4). Skin-test responses were measured for the protein cocktail compromising ESAT-6, CFP-10, MPB70 and MPB83 (10µg for each component) and for the SICCT-test [(PPD-B)-(PPD-A)] in naive TB-free (n=19) and BCG vaccinated (n=20) cattle. In Figure 4, responses measured at 72 hours for each animal are represented by an open circle, the horizontal line provides the mean (± SEM) with results expressed as the difference in skin thickness (mm) between the pre and post skin-test readings. Whilst all SICCT responses were also negative, measurable PPD-A biased responses did indicate some sensitisation of these animals by environmental mycobacteria. Reactions were additionally measured at 96 and 120 hours. For all further experiments, reactions were only recorded at 72 hours post-test.

The DIVA potential of the protein cocktail was evaluated using 3-month old calves that had been neo-natally vaccinated with BCG at 4-6 weeks of age (n=20). In these calves, only one animal failed to induce a SICCT PPD-B biased skin-test, response confirming the strong tuberculin skin-test sensitisation of BCG vaccination (Figure 4). In contrast, the defined protein cocktail did not induce a response in any of these vaccinates. Protein cocktail and SICCT responses measured in either the naturally infected or BCG vaccinated cattle were used to generate ROC curves to provide estimates of DIVA skin-test sensitivity and specificity. Table 1 summarises the sensitivity and specificity estimates for a SICCT cut-off of >2mm and >4mm since these represent operational 'severe' and 'standard' test cut-offs used in UK surveillance operations (http://www.defra.gov.uk/food-farm/animals/diseases/tb/, accessed 18 April 2011). Also shown are the performance figures for SICCT and protein cocktail test cut-offs that provide 100% DIVA specificity for the respective tests.

**Table 1 DIVA sensitivity and specificity ROC estimates for SICCT and defined protein cocktail based skin-tests**

| Diagnostic Antigen | Cut-off (mm) | Sensitivity^{a} (%) | Specificity^{b} (%) | Area under curve | 95% CI |
|---|---|---|---|---|---|
| SICCT (PPD B-A) | > 2 | 100 | 15.0 | 0.899 | 0.817-0.981 |
| SICCT (PPD B-A) | > 4 | 94.1 | 40.0 | 0.899 | 0.817-0.981 |
| SICCT (PPD B-A) | > 10 | 55.9 | 100 | 0.899 | 0.817-0.981 |
| Protein cocktail^{c} | > 1 | 73.6 | 100 | 0.882 | 0.791-0.974 |

| | | | | | |
|---|---|---|---|---|---|
| ^{a} Sensitivity determined using diseased confirmed SICCT 'reactor' naturally infected cattle (n=34). ^{b} Specificity determined using disease free cattle, BCG-vaccinated calves (n=20). ^{c} Protein cocktail comprising 10µg each of ESAT-6 (SEQ ID NO:36), CFP-10 (SEQ ID NO:35), MPB70 (SEQ ID NO:39) and MPB83 (SEQ ID NO:37). | | | | | |

### Optimisation of defined skin-test reagents including the use of Rv3615c and synthetic polypeptides

*In vitro* assessment of the capacity of the constituent protein cocktail antigens to induce IFN-γ suggested the immunodominance of ESAT-6 and CFP-10 (Figure 2). Therefore, the additive benefit of the inclusion of MPB70 and MPB83 to a cocktail already containing ESAT-6 and CFP-10 was investigated. In a group of 12 previously untested naturally infected cattle, all of which induced positive SICCT responses (data not shown), 6/12 cattle induced a response to the cocktail containing all 4 antigens (1-6mm responses) whilst only 3 of these cattle responded to the cocktail containing only ESAT-6 and CFP-10 (2, 3 and 6mm responses). Although the difference in responder frequency was not significant, the result did indicate an additive benefit of including MPB70 and/or MPB83 to the cocktail.

To determine if the inclusion of both MPB83 and MPB70 was required for optimal skin responses, protein cocktails formulated with and without MPB70 were tested in a further 13 naturally infected cattle. Additionally, this experiment was used as an opportunity to evaluate two novel defined antigen formulations. Firstly, a protein cocktail containing Rv3615c, a recently identified DIVA antigen which can identify *M. bovis* infected cattle that are unresponsive to ESAT-6 or CFP-10 (WO2009/060184 and Sidders et al. (2008) Infect. Immun. vol. 76 pp 3932-9). Secondly, a synthetic polypeptide cocktail comprising overlapping polypeptides of ESAT-6 and CFP-10. The results are shown in Figure 5, in which responses measured at 72 hours for each animal are represented by an open circle, the horizontal line provides the mean (± SEM) with results expressed as the difference in skin thickness (mm) between the pre and post skin-test readings. Statistical difference between responses induced by the defined protein and polypeptide cocktails was determined using ANOVA (*** p<0.001).

The inclusion of MPB70 demonstrated no improvement in the responder frequency or magnitude of skin-reactions in these cattle, compared with those responses induced by the ESAT6, CFP-10 and MPB83 containing cocktail (Figure 5). In contrast, the addition of Rv3615c increased responder frequency from 10/13 to 13/13 and also resulted in significantly stronger responses (Figure 5). Protein combinations of ESAT-6 (E6), CFP-10 (C10), MPB70, MPB83 (83) and/or Rv3615c were all tested at 10µg per constituent protein.

The ESAT-6 and CFP-10 (E6/C10) polypeptide cocktail included 21 polypeptides (SEQ ID NOs:1-21) at a concentration of 10µg per polypeptide. As Figure 5 shows, the ESAT-6/CFP-10 polypeptide cocktail induces responses in 11/13 cattle thus demonstrating their potential as skin-test antigens.

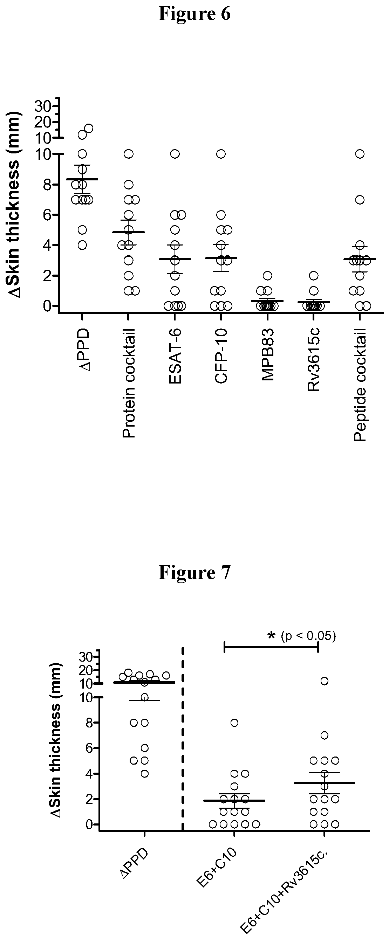

The skin-test contribution of the individual antigens present in the protein cocktail comprising ESAT-6, CFP-10, MPB83 and Rv3615c was then investigated. The evaluation of synthetic polypeptide combinations was extended by testing a cocktail of polypeptides derived from CFP-10 (SEQ ID NOs:1-10), ESAT-6 (SEQ ID NOs:11-21), MPB83 (SEQ ID NO:22) and Rv3615c (SEQ ID NOs:31, 32 & 34). These reagents were tested in 12 reactor cattle that were selected from those used in the previous 2 experiments. All 9 animals that responded to any single antigen recognised CFP-10 (cut-off for positivity >1mm) whilst 7, 3, and 2 responded to ESAT-6, MPB83 and Rv3615c respectively (Figure 6). In Figure 6, reactions measured at 72 hours for each animal are represented by an open circle, the horizontal line provides the mean (± SEM) with results expressed as the difference in skin thickness (mm) between the pre and post skin-test readings.

The combined protein cocktail provided maximal responder frequency and reaction size, including in 3 animals that failed to respond to any of the individual proteins. Encouragingly, the polypeptide cocktail induced responses in 10/12 of these cattle as shown in Figure 6.

The specificity of the protein combination of ESAT-6, CFP-10, MPB83 and Rv3615c, as used above, was evaluated in 14 new naïve cattle and 19 of the BCG neo-natally vaccinated calves previously tested. The protein cocktail did not induce responses in any of these naive or vaccinated cattle. Whilst 16/19 of the BCG vaccinates still demonstrated a PPD-B biased IFN-y response at this time ([PPD-B]-[PPD-A] >0.1 OD₄₅₀ₙₘ, data not shown), it should be noted however that only 3/19 of the BCG vaccinates still elicited PPD-B biased skin reaction at this test, due to the extended period of nearly 12 months following neo-natal vaccination at this time (data not shown). Therefore, the use of the cocktail provides a more specific assay even after an extended period of up to 12 months post-vaccination.

The inventors also evaluated the skin-test performance of an ESAT-6/CFP-10 based diagnostic polypeptide cocktail that additionally includes 11 Rv3615c polypeptides disclosed in WO2009/060184 (SEQ ID NOs:23-33).

These 11 polypeptides provide fully overlapping coverage of residues 1-100 of the Rv3615c protein sequence. Therefore, the polypeptides in the cocktail were SEQ ID NOs:1-21 and 23-33. Skin-test responses were measured in SICCT-test positive (reactor) cattle (n=15). These animals were previously recruited from UK farms during routine TB surveillance operations. Skin reactions were measured 3 days following intradermal injection of antigens (100µl injection volume). Each of the polypeptide cocktails contained an administered dose of 10µg of each component polypeptide.

The increase in skin thickness 3 days following administration of antigens is shown in Figure 7. All animals had SICCT positive skin-test. The addition of the 11 Rv3615c polypeptides to an ESAT-6/CFP-10 polypeptide-based cocktail resulted in significantly stronger skin-test responses compared with the cocktail containing polypeptides of ESAT6 and CFP10 alone (p=0.018, paired t-test).

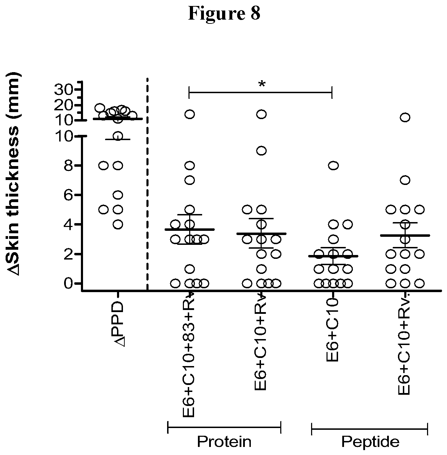

Finally, the inventors determined that the optimal combination of epitope polypeptides (proteins or epitope polypeptide fragments) was ESAT-6, CFP-10 and Rv3615c. As can be seen in Figure 8, no additional sensitivity of the skin test was achieved by the further inclusion of the protein MPB83.

### Discussion

Previous investigations on the use of defined bovine skin-test reagents suggested that either immunomodulating reagents (Whelan et al. (2003) Infect. Immun. vol. 71 pp 6420-5) or high antigen doses (≥400ug) (Pollock et al. (2003) J. Clin. Microbiol. vol. 41 pp 1856-60) might be required to provide sensitive skin-responses. It is therefore surprising that the current work demonstrated antigen specific skin-reactions in 78% of SICCT reactor cattle when using a protein-antigen combination of ESAT-6, CFP-10, MPB70 and MPB83, using an administered concentration of 10µg per protein Importantly, this is a dose considered to be realistic for practical field applications. Furthermore, this skin-test cocktail was highly specific, eliciting no response in either naive or BCG vaccinated calves. This is the first time that the DIVA potential of defined skin-test antigens has been demonstrated in BCG vaccinated/*M*. *bovis* infected cattle.

Assessment of the *in vitro* recognition of the individual antigens in the initial protein cocktail showed that MPB70 and MPB83 had poor IFN-γ inducing capacity in comparison with ESAT-6 and CFP-10. Therefore, despite their sub-dominant *in vitro* immunogenicity in cattle, it is surprising that their inclusion in the skin-test cocktail increased the skin-test responder frequency compared with using only ESAT-6 and CFP-10. In mice, it has been shown that during a DTH (delayed-type hypersensitivity) response, there must first be an early initiation phase which is required to recruit antigen specific T-cells which then propagate the classical late phase inflammatory response (van Loveren et al. (1983) J. Exp. Med. vol. 157 pp 1604-17). Furthermore, the antigens that induce the initiation and effector stages can be different (Ptak et al. (1986) J. Immunol. vol. 136 pp 1564-70). Therefore, without being bound by theory, it is possible that sub-dominant effector T-cell antigens such as MPB70 and MPB83 might still have a role in promoting reaction-initiation, thereby helping to elicit a better response to dominant effector antigens such as ESAT-6 and CFP-10.

Further optimisation of the skin-test protein cocktail demonstrated that the inclusion of MPB70 had no additional benefit over the use of MPB83. However, the addition of Rv3615c to a protein cocktail containing ESAT-6, CFP-10 and MPB83 did result in a significant improvement in skin-test responses and proved to be the most optimal antigen combination tested. The diagnostic potential of Rv3615c has only recently been identified, having been found to be recognised by infected cattle which did not respond to either ESAT-6 or CFP-10 (WO2009/060184 and Sidders et al. (2008) Infect. Immun. vol. 76 pp 3932-9). The present demonstration that it can also contribute to improved skin-test responses without compromising specificity further confirms its diagnostic importance. Furthermore, the inventors have shown that the advantages described herein are obtained using a combination of ESAT-6, CFP-10 and Rv3615c, with inclusion of MPB83 not being necessary for optimal skin test sensitivity when all three of the proteins (or epitope polypeptide fragments) are included.

An important practical advantage of synthetic polypeptides compared to recombinant proteins as diagnostic antigens is that, being chemically synthesised, quality control is more easily standardised. The practical application of synthetic polypeptides as bovine TB DIVA reagents using IFN-γ based blood assays (Vordermeier et al. (2001) Clin. Diagn. Lab. Immunol. vol. 8 pp 571-8) has previously been demonstrated and, similarly, they have also been applied for human TB diagnosis (Arend et al. (2000) J. Infect. Dis. vol. 181 pp 1850-4; Lalvani et al. (2001) J. Infect. Dis. vol. 183 pp 469-77). However, this is the first report demonstrating the potential of these protein fragment polypeptides as TB skin-test antigens in cattle. This data provides a basis for future optimisation and improvement of a polypeptide based skin-test.

In considering why the results of previous ESAT-6 based cattle skin-test studies looked less promising than the current data (Pollock et al. (2003) J. Clin. Microbiol. vol. 41 pp 1856-60; Whelan et al. (2003) Infect. Immun. vol 71 pp 6420-5), one explanation is likely to be the inclusion of CFP-10, which has not previously been evaluated as a bovine skin-test antigen. Notably, skin-test responses induced by the individual protein antigens showed CFP-10 to be the most potent defined antigen. Furthermore, the use of antigen combinations demonstrated clear sensitivity benefits in the current study and will also have contributed to the improved responses compared with the previous use of ESAT-6 alone (Pollock et al. (2003) J. Clin. Microbiol. vol. 41 pp 1856-60; Whelan et al. (2003) Infect. Immun. vol 71 pp 6420-5). When comparing cellular responses induced by recombinant protein antigens, the presence of contamination endotoxin should also be considered. However, it is unlikely that immunomodulation from possible endotoxin contamination can explain the potent responses to the protein antigens in the current study, since skin-reactions were also induced by the synthetic polypeptides which are endotoxin-free.

Pollock *et al.* also demonstrated that PPD-induced bovine skin reactions were maximal at 72 hours whilst the response to ESAT-6 was often greatest at 96 hours (Pollock et al. (2003) J. Clin. Microbiol. vol. 41 pp 1856-60). The present data confirmed 72 hours to be optimal for the measurement of the SICCT response (Figure 1) and increases in the protein-cocktail responder frequency were also observed when reactions were read at 96 or 120 hours, such that responder frequency increased from 29/37, to 31/37 and then 32/37 for the 72, 96 and 120 hour time-points respectively. However, the increase in responder frequency was not significant (data not shown) and there was no increase in the magnitude of comparative responses. Since highly specific reactions were observed in the majority of animals at 72 hours using the optimised defined cocktails, measuring responses at this time point has the practical benefit of allowing the option of testing defined antigens and PPD in parallel and then reading the reaction on the same day.

## Claims

1. A skin test diagnostic reagent in the form of a sterile injectable preparation, comprising:
a. the CFP-10 epitope polypeptides consisting of amino acid sequences SEQ ID NOs:1-10;
b. the ESAT-6 epitope polypeptides consisting of amino acid sequences SEQ ID NOs:11-21; and
c. the Rv3615c epitope polypeptides consisting of amino acid sequences SEQ ID NOs:23-34.

2. A diagnostic reagent according to claim 1 which comprises one or more of the polypeptides having amino acid sequences SEQ ID NOs:35, 36 and 38.

3. A diagnostic reagent according to any preceding claim which further comprises at least one MPB83 epitope polypeptide.

4. A diagnostic reagent according to claim 3 which comprises a polypeptide having amino acid sequence SEQ ID NO:37 and/or which comprises a polypeptide having amino acid sequence SEQ ID NO:22.

5. A diagnostic reagent according to any preceding claim comprising one or more fusion proteins.

6. Diagnostic reagent according to any preceding claim for use in a method of detecting *Mycobacterium bovis* or *Mycobacterium tuberculosis* infection in an animal using a skin test.

7. Diagnostic reagent according to any preceding claim for use in a method of diagnosing infection of an animal by *Mycobacterium bovis* or *Mycobacterium tuberculosis,* the method comprising the steps of conducting a skin test on the animal using the diagnostic reagent and correlating a positive skin test result with infection of the animal by *Mycobacterium bovis* or *Mycobacterium tuberculosis.*

8. A diagnostic kit comprising a diagnostic reagent according to any preceding claim.

9. A diagnostic kit according to claim 8 wherein the diagnostic reagent is in liquid form and included in at least one aliquot of 0.05-0.15ml containing 1-15µg of each polypeptide contained in the diagnostic reagent.

10. A diagnostic kit according to claim 9 wherein each aliquot is contained in a disposable injection device.

11. A diagnostic kit according to any of claims 8-10 in which the diagnostic reagent is able to detect a *M*. *bovis* or *M. tuberculosis* infection in a mammal.

12. A diagnostic kit according to claim 11 in which the diagnostic reagent is able to differentiate between an *M. bovis-* or *M. tuberculosis*-infected mammal and a mammal vaccinated against infection by *M. bovis* or *M. tuberculosis.*

## Patentansprüche

1. Diagnostisches Reagens für Hauttests in Form einer sterilen injizierbaren Zubereitung, umfassend:
a. die aus den Aminosäuresequenzen SEQ ID NO:1-10 bestehenden Polypeptide eines CFP-10-Epitops,
b. die aus den Aminosäuresequenzen SEQ ID NO:11-21 bestehenden Polypeptide eines ESAT-6-Epitops und
c. die aus den Aminosäuresequenzen SEQ ID NO:23-34 bestehenden Polypeptide eines Rv3615c-Epitops.

2. Diagnostisches Reagens nach Anspruch 1, welches eines oder mehrere der Polypeptide mit den Aminosäuresequenzen SEQ ID NO:35, 36 und 38 umfasst.

3. Diagnostisches Reagens nach einem der vorhergehenden Ansprüche, welches ferner mindestens ein Polypeptid eines MPB83-Epitops umfasst.

4. Diagnostisches Reagens nach Anspruch 3, welches ein Polypeptid mit der Aminosäuresequenz SEQ ID NO:37 und/oder welches ein Polypeptid mit der Aminosäuresequenz SEQ ID NO:22 umfasst.

5. Diagnostisches Reagens nach einem der vorhergehenden Ansprüche, umfassend ein oder mehrere Fusionsproteine.

6. Diagnostisches Reagens nach einem der vorhergehenden Ansprüche zur Verwendung in einem Verfahren zum Nachweisen einer Infektion mit *Mycobacterium bovis* oder *Mycobacterium tuberculosis* bei einem Tier unter Verwendung eines Hauttests.

7. Diagnostisches Reagens nach einem der vorhergehenden Ansprüche zur Verwendung in einem Verfahren zum Diagnostizieren einer Infektion eines Tiers mit *Mycobacterium bovis* oder *Mycobacterium tuberculosis,* wobei das Verfahren die Schritte des Durchführens eines Hauttests an dem Tier unter Verwendung des diagnostischen Reagens sowie des Korrelierens eines positiven Hauttestergebnisses mit einer Infektion des Tiers mit *Mycobacterium bovis* oder *Mycobacterium tuberculosis* umfasst.

8. Diagnostisches Kit, umfassend ein diagnostisches Reagens nach einem der vorhergehenden Ansprüche.

9. Diagnostisches Kit nach Anspruch 8, wobei das diagnostische Reagens in flüssiger Form vorliegt und in mindestens einem Aliquot von 0,05 bis 0,15 ml enthalten ist, welches 1 bis 15 µg von jedem in dem diagnostischen Reagens enthaltenen Polypeptid enthält.

10. Diagnostisches Kit nach Anspruch 9, wobei jedes Aliquot in einer Einweg-Injektionsvorrichtung enthalten ist.

11. Diagnostisches Kit nach einem der Ansprüche 8 bis 10, bei welchem das diagnostische Reagens in der Lage ist, eine Infektion mit *M*. *bovis* oder *M. tuberculosis* bei einem Säuger nachzuweisen.

12. Diagnostisches Kit nach Anspruch 11, bei welchem das diagnostische Reagens in der Lage ist, zwischen einem mit *M. bovis* oder *M*. *tuberculosis* infizierten Säuger und einem gegen Infektion mit *M*. *bovis* oder *M*. *tuberculosis* geimpften Säuger zu unterscheiden.

## Revendications

1. Réactif de diagnostic pour test cutané sous forme d'une préparation injectable stérile, comprenant:
a.les polypeptides à épitope(s) de CFP-10 consistant en les séquences d'acides aminés SEQ ID NO: 1-10;
b.les polypeptides à épitope(s) de ESAT-6 consistant en les séquences d'acides aminés SEQ ID NO: 11-21; et
c.les polypeptides à épitope(s) de Rv3615c consistant en les séquences d'acides aminés SEQ ID NO: 23-34.

2. Réactif de diagnostic selon la revendication 1 qui comprend un ou plusieurs des polypeptides ayant les séquences d'acides aminés SEQ ID NO: 35, 36 et 38.

3. Réactif de diagnostic selon l'une quelconque des revendications précédentes qui comprend en outre au moins un polypeptide à épitope(s) de MPB83.

4. Réactif de diagnostic selon la revendication 3 qui comprend un polypeptide ayant la séquence d'acides aminés SEQ ID NO: 37 et/ou qui comprend un polypeptide ayant la séquence d'acides aminés SEQ ID NO: 22.

5. Réactif de diagnostic selon l'une quelconque des revendications précédentes comprenant une ou plusieurs protéines de fusion.

6. Réactif de diagnostic selon l'une quelconque des revendications précédentes destiné à être utilisé dans un procédé de détection d'une infection par *Mycobacterium bovis* ou *Mycobacterium tuberculosis* chez un animal au moyen d'un test cutané.

7. Réactif de diagnostic selon l'une quelconque des revendications précédentes destiné à être utilisé dans un procédé de diagnostic d'une infection d'un animal par *Mycobacterium bovis* ou *Mycobacterium tuberculosis,* le procédé comprenant les étapes de réalisation d'un test cutané sur l'animal au moyen du réactif de diagnostic et de corrélation d'un résultat de test cutané positif avec une infection de l'animal par *Mycobacterium bovis* ou *Mycobacterium tuberculosis.*

8. Kit de diagnostic comprenant un réactif de diagnostic selon l'une quelconque des revendications précédentes.

9. Kit de diagnostic selon la revendication 8 où le réactif de diagnostic est sous forme liquide et inclus dans au moins une portion aliquote de 0,05-0,15 ml contenant 1-15 µg de chaque polypeptide contenu dans le réactif de diagnostic.

10. Kit de diagnostic selon la revendication 9 où chaque portion aliquote est contenue dans un dispositif d'injection jetable.

11. Kit de diagnostic selon l'une quelconque des revendications 8-10 dans lequel le réactif de diagnostic est capable de détecter une infection par *M*. *bovis* ou *M. tuberculosis* chez un mammifère.

12. Kit de diagnostic selon la revendication 11 dans lequel le réactif de diagnostic est capable de différencier entre un mammifère infecté par *M. bovis* ou *M. tuberculosis* et un mammifère vacciné contre une infection par *M. bovis* ou *M. tuberculosis.*


### SEQUENCE LISTING

<110> The Secretary of State for Environment, Food and Rural Affairs acting through the Veterinary Laboratories Agency Whelan, Adam O
   Vordermeier, Hanns M
<120> Antigens
<130> RT/P1761PC00
<150> GB1007075.3
   <151> 2010-04-28
<160> 39
<170> PatentIn version 3.5
<210> 1
   <211> 18
   <212> PRT
   <213> Artificial
<220>
   <223> Fragment of full length protein
<400> 1
   Met Ala Glu Met Lys Thr Asp Ala Ala Thr Leu Ala Gln Glu Ala Gly
   1               5                   10                  15
   Asn Phe
<210> 2
   <211> 16
   <212> PRT
   <213> Artificial
<220>
   <223> Fragment of full length protein
<400> 2
   Gln Glu Ala Gly Asn Phe Glu Arg Ile Ser Gly Asp Leu Lys Thr Gln
   1               5                   10                  15
<210> 3
   <211> 18
   <212> PRT
   <213> Artificial
<220>
   <223> Fragment of full length protein
<400> 3
   Glu Arg Ile Ser Gly Asp Leu Lys Thr Gln Ile Asp Gln Val Glu Ser
   1               5                   10                  15
   Thr Ala
<210> 4
   <211> 17
   <212> PRT
   <213> Artificial
<220>
   <223> Fragment of full length protein
<400> 4
   Ile Asp Gln Val Glu Ser Thr Ala Gly Ser Leu Gln Gly Gln Trp Arg
   1               5                   10                  15
   Gly
<210> 5
   <211> 18
   <212> PRT
   <213> Artificial
<220>
   <223> Fragment of full length protein
<400> 5
   Gly Ser Leu Gln Gly Gln Trp Arg Gly Ala Ala Gly Thr Ala Ala Gln
   1               5                   10                  15
   Ala Ala
<210> 6
   <211> 18
   <212> PRT
   <213> Artificial
<220>
   <223> Fragment of full length protein
<400> 6
   Ala Gly Thr Ala Ala Gln Ala Ala Val Val Arg Phe Gln Glu Ala Ala
   1               5                   10                  15
   Asn Lys
<210> 7
   <211> 18
   <212> PRT
   <213> Artificial
<220>
   <223> Fragment of full length protein
<400> 7
   Val Val Arg Phe Gln Glu Ala Ala Asn Lys Gln Lys Gln Glu Leu Asp
   1               5                   10                  15
   Glu Ile
<210> 8
   <211> 20
   <212> PRT
   <213> Artificial
<220>
   <223> Fragment of full length protein
<400> 8
   Gln Lys Gln Glu Leu Asp Glu Ile Ser Thr Asn Ile Arg Gln Ala Gly
   1               5                   10                  15
   Val Gln Tyr Ser
               20
<210> 9
   <211> 18
   <212> PRT
   <213> Artificial
<220>
   <223> Fragment of full length protein
<400> 9
   Asn Ile Arg Gln Ala Gly Val Gln Tyr Ser Arg Ala Asp Glu Glu Gln
   1 5 10 15
   Gln Gln
<210> 10
   <211> 16
   <212> PRT
   <213> Artificial
<220>
   <223> Fragment of full length protein
<400> 10
   Arg Ala Asp Glu Glu Gln Gln Gln Ala Leu Ser Ser Gln Met Gly Phe
   1 5 10 15
<210> 11
   <211> 16
   <212> PRT
   <213> Artificial
<220>
   <223> Fragment of full length protein
<400> 11
   Met Thr Glu Gln Gln Trp Asn Phe Ala Gly Ile Glu Ala Ala Ala Ser
   1 5 10 15
<210> 12
   <211> 16
   <212> PRT
   <213> Artificial
<220>
   <223> Fragment of full length protein
<400> 12
   Ala Gly Ile Glu Ala Ala Ala Ser Ala Ile Gln Gly Asn Val Thr Ser
   1 5 10 15
<210> 13
   <211> 16
   <212> PRT
   <213> Artificial
<220>
   <223> Fragment of full length protein
<400> 13
   Ala Ile Gln Gly Asn Val Thr Ser Ile His Ser Leu Leu Asp Glu Gly
   1 5 10 15
<210> 14
   <211> 16
   <212> PRT
   <213> Artificial
<220>
   <223> Fragment of full length protein
<400> 14
   Ile His Ser Leu Leu Asp Glu Gly Lys Gln Ser Leu Thr Lys Leu Ala
   1               5                   10                  15
<210> 15
   <211> 16
   <212> PRT
   <213> Artificial
<220>
   <223> Fragment of full length protein
<400> 15
   Lys Gln Ser Leu Thr Lys Leu Ala Ala Ala Trp Gly Gly Ser Gly Ser
   1               5                   10                  15
<210> 16
   <211> 16
   <212> PRT
   <213> Artificial
<220>
   <223> Fragment of full length protein
<400> 16
   Ala Ala Trp Gly Gly Ser Gly Ser Glu Ala Tyr Gln Gly Val Gln Gln
   1               5                   10                  15
<210> 17
   <211> 16
   <212> PRT
   <213> Artificial
<220>
   <223> Fragment of full length protein
<400> 17
   Glu Ala Tyr Gln Gly Val Gln Gln Lys Trp Asp Ala Thr Ala Thr Glu
   1               5                   10                  15
<210> 18
   <211> 16
   <212> PRT
   <213> Artificial
<220>
   <223> Fragment of full length protein
<400> 18
   Lys Trp Asp Ala Thr Ala Thr Glu Leu Asn Asn Ala Leu Gln Asn Leu
   1               5                   10                  15
<210> 19
   <211> 16
   <212> PRT
   <213> Artificial
<220>
   <223> Fragment of full length protein
<400> 19
   Leu Asn Asn Ala Leu Gln Asn Leu Ala Arg Thr Ile Ser Glu Ala Gly
   1               5                   10                  15
<210> 20
   <211> 16
   <212> PRT
   <213> Artificial
<220>
   <223> Fragment of full length protein
<400> 20
   Ala Arg Thr Ile Ser Glu Ala Gly Gln Ala Met Ala Ser Thr Glu Gly
   1               5                   10                  15
<210> 21
   <211> 15
   <212> PRT
   <213> Artificial
<220>
   <223> Fragment of full length protein
<400> 21
   Gln Ala Met Ala Ser Thr Glu Gly Asn Val Thr Gly Met Phe Ala
   1               5                   10                  15
<210> 22
   <211> 20
   <212> PRT
   <213> Artificial
<220>
   <223> Fragment of full length protein
<400> 22
   Gly Leu Val Cys Gly Gly Val His Thr Ala Asn Ala Thr Val Tyr Met
   1 5 10 15
   Ile Asp Thr Val
   20
<210> 23
   <211> 20
   <212> PRT
   <213> Artificial
<220>
   <223> Fragment of full length protein
<400> 23
   Met Thr Glu Asn Leu Thr Val Gln Pro Glu Arg Leu Gly Val Leu Ala
   1 5 10 15
   Ser His His Asp
   20
<210> 24
   <211> 20
   <212> PRT
   <213> Artificial
<220>
   <223> Fragment of full length protein
<400> 24
   Pro Glu Arg Leu Gly Val Leu Ala Ser His His Asp Asn Ala Ala Val
   1 5 10 15
   Asp Ala Ser Ser
   20
<210> 25
   <211> 20
   <212> PRT
   <213> Artificial
<220>
   <223> Fragment of full length protein
<400> 25
   Ser His His Asp Asn Ala Ala Val Asp Ala Ser Ser Gly Val Glu Ala
   1 5 10 15
   Ala Ala Gly Leu
   20
<210> 26
   <211> 20
   <212> PRT
   <213> Artificial
<220>
   <223> Fragment of full length protein
<400> 26
   Asp Ala Ser Ser Gly Val Glu Ala Ala Ala Gly Leu Gly Glu Ser Val
   1               5                   10                  15
   Ala Ile Thr His
               20
<210> 27
   <211> 20
   <212> PRT
   <213> Artificial
<220>
   <223> Fragment of full length protein
<400> 27
   Ala Ala Gly Leu Gly Glu Ser Val Ala Ile Thr His Gly Pro Tyr Cys
   1               5                   10                  15
   Ser Gln Phe Asn
               20
<210> 28
   <211> 20
   <212> PRT
   <213> Artificial
<220>
   <223> Fragment of full length protein
<400> 28
   Ala Ile Thr His Gly Pro Tyr Cys Ser Gln Phe Asn Asp Thr Leu Asn
   1               5                   10                  15
   Val Tyr Leu Thr
               20
<210> 29
   <211> 20
   <212> PRT
   <213> Artificial
<220>
   <223> Fragment of full length protein
<400> 29
   Ser Gln Phe Asn Asp Thr Leu Asn Val Tyr Leu Thr Ala His Asn Ala
   1               5                   10                  15
   Leu Gly Ser Ser
               20
<210> 30
   <211> 20
   <212> PRT
   <213> Artificial
<220>
   <223> Fragment of full length protein
<400> 30
   Val Tyr Leu Thr Ala His Asn Ala Leu Gly Ser Ser Leu His Thr Ala
   1               5                   10                  15
   Gly Val Asp Leu
               20
<210> 31
   <211> 20
   <212> PRT
   <213> Artificial
<220>
   <223> Fragment of full length protein
<400> 31
   Leu Gly Ser Ser Leu His Thr Ala Gly Val Asp Leu Ala Lys Ser Leu
   1               5                   10                  15
   Arg Ile Ala Ala
               20
<210> 32
   <211> 20
   <212> PRT
   <213> Artificial
<220>
   <223> Fragment of full length protein
<400> 32
   Gly Val Asp Leu Ala Lys Ser Leu Arg Ile Ala Ala Lys Ile Tyr Ser
   1               5                   10                  15
   Glu Ala Asp Glu
               20
<210> 33
   <211> 20
   <212> PRT
   <213> Artificial
<220>
   <223> Fragment of full length protein
<400> 33
   Arg Ile Ala Ala Lys Ile Tyr Ser Glu Ala Asp Glu Ala Trp Arg Lys
   1               5                   10                  15
   Ala Ile Asp Gly
               20
<210> 34
   <211> 20
   <212> PRT
   <213> Artificial
<220>
   <223> Fragment of full length protein
<400> 34
   Ala Lys Ile Tyr Ser Glu Ala Asp Glu Ala Trp Arg Lys Ala Ile Asp
   1               5                   10                  15
   Gly Leu Phe Thr
               20
<210> 35
   <211> 100
   <212> PRT
   <213> Mycobacterium bovis
<400> 35
   Met Ala Glu Met Lys Thr Asp Ala Ala Thr Leu Ala Gln Glu Ala Gly
   1               5                   10                  15
   Asn Phe Glu Arg Ile Ser Gly Asp Leu Lys Thr Gln Ile Asp Gln Val
               20                  25                  30
   Glu Ser Thr Ala Gly Ser Leu Gln Gly Gln Trp Arg Gly Ala Ala Gly
           35                  40                  45
   Thr Ala Ala Gln Ala Ala Val Val Arg Phe Gln Glu Ala Ala Asn Lys
       50                  55                  60
   Gln Lys Gln Glu Leu Asp Glu Ile Ser Thr Asn Ile Arg Gln Ala Gly
   65                  70                  75                  80
   Val Gln Tyr Ser Arg Ala Asp Glu Glu Gln Gln Gln Ala Leu Ser Ser
                   85                  90                  95
   Gln Met Gly Phe
               100
<210> 36
   <211> 95
   <212> PRT
   <213> Mycobacterium bovis
<400> 36
   Met Thr Glu Gln Gln Trp Asn Phe Ala Gly Ile Glu Ala Ala Ala Ser
   1               5                   10                  15
   Ala Ile Gln Gly Asn Val Thr Ser Ile His Ser Leu Leu Asp Glu Gly
               20                  25                  30
   Lys Gln Ser Leu Thr Lys Leu Ala Ala Ala Trp Gly Gly Ser Gly Ser
           35                  40                  45
   Glu Ala Tyr Gln Gly Val Gln Gln Lys Trp Asp Ala Thr Ala Thr Glu
       50                  55                  60
   Leu Asn Asn Ala Leu Gln Asn Leu Ala Arg Thr Ile Ser Glu Ala Gly
   65                  70                  75                  80
   Gln Ala Met Ala Ser Thr Glu Gly Asn Val Thr Gly Met Phe Ala
                   85                  90                  95
<210> 37
   <211> 220
   <212> PRT
   <213> Mycobacterium bovis
<400> 37
   Met Ile Asn Val Gln Ala Lys Pro Ala Ala Ala Ala Ser Leu Ala Ala
   1               5                   10                  15
   Ile Ala Ile Ala Phe Leu Ala Gly Cys Ser Ser Thr Lys Pro Val Ser
               20                  25                  30
   Gln Asp Thr Ser Pro Lys Pro Ala Thr Ser Pro Ala Ala Pro Val Thr
           35                  40                  45
   Thr Ala Ala Met Ala Asp Pro Ala Ala Asp Leu Ile Gly Arg Gly Cys
       50                  55                  60
   Ala Gln Tyr Ala Ala Gln Asn Pro Thr Gly Pro Gly Ser Val Ala Gly
   65                  70                  75                  80
   Met Ala Gln Asp Pro Val Ala Thr Ala Ala Ser Asn Asn Pro Met Leu
                   85                  90                  95
   Ser Thr Leu Thr Ser Ala Leu Ser Gly Lys Leu Asn Pro Asp Val Asn
               100                 105                 110
   Leu Val Asp Thr Leu Asn Gly Gly Glu Tyr Thr Val Phe Ala Pro Thr
           115                 120                 125
   Asn Ala Ala Phe Asp Lys Leu Pro Ala Ala Thr Ile Asp Gln Leu Lys
       130                 135                 140
   Thr Asp Ala Lys Leu Leu Ser Ser Ile Leu Thr Tyr His Val Ile Ala
   145                 150                 155                 160
   Gly Gln Ala Ser Pro Ser Arg Ile Asp Gly Thr His Gln Thr Leu Gln
                   165                 170                 175
   Gly Ala Asp Leu Thr Val Ile Gly Ala Arg Asp Asp Leu Met Val Asn
               180                 185                 190
   Asn Ala Gly Leu Val Cys Gly Gly Val His Thr Ala Asn Ala Thr Val
           195                 200                 205
   Tyr Met Ile Asp Thr Val Leu Met Pro Pro Ala Gln
       210                 215                 220
<210> 38
   <211> 103
   <212> PRT
   <213> Mycobacterium bovis
<400> 38
   Met Thr Glu Asn Leu Thr Val Gln Pro Glu Arg Leu Gly Val Leu Ala
   1               5                   10                  15
   Ser His His Asp Asn Ala Ala Val Asp Ala Ser Ser Gly Val Glu Ala
               20                  25                  30
   Ala Ala Gly Leu Gly Glu Ser Val Ala Ile Thr His Gly Pro Tyr Cys
           35                  40                  45
   Ser Gln Phe Asn Asp Thr Leu Asn Val Tyr Leu Thr Ala His Asn Ala
       50                  55                  60
   Leu Gly Ser Ser Leu His Thr Ala Gly Val Asp Leu Ala Lys Ser Leu
   65                  70                  75                  80
   Arg Ile Ala Ala Lys Ile Tyr Ser Glu Ala Asp Glu Ala Trp Arg Lys
                   85                  90                  95
   Ala Ile Asp Gly Leu Phe Thr
               100
<210> 39
   <211> 193
   <212> PRT
   <213> Mycobacterium bovis
<400> 39
   Met Lys Val Lys Asn Thr Ile Ala Ala Thr Ser Phe Ala Ala Ala Gly
   1               5                   10                  15
   Leu Ala Ala Leu Ala Val Ala Val Ser Pro Pro Ala Ala Ala Gly Asp
               20                  25                  30
   Leu Val Gly Pro Gly Cys Ala Glu Tyr Ala Ala Ala Asn Pro Thr Gly
           35                  40                  45
   Pro Ala Ser Val Gln Gly Met Ser Gln Asp Pro Val Ala Val Ala Ala
       50                  55                  60
   Ser Asn Asn Pro Glu Leu Thr Thr Leu Thr Ala Ala Leu Ser Gly Gln
   65                  70                  75                  80
   Leu Asn Pro Gln Val Asn Leu Val Asp Thr Leu Asn Ser Gly Gln Tyr
                   85                  90                  95
   Thr Val Phe Ala Pro Thr Asn Ala Ala Phe Ser Lys Leu Pro Ala Ser
               100                 105                 110
   Thr Ile Asp Glu Leu Lys Thr Asn Ser Ser Leu Leu Thr Ser Ile Leu
           115                 120                 125
   Thr Tyr His Val Val Ala Gly Gln Thr Ser Pro Ala Asn Val Val Gly
       130                 135                 140
   Thr Arg Gln Thr Leu Gln Gly Ala Ser Val Thr Val Thr Gly Gln Gly
   145                 150                 155                 160
   Asn Ser Leu Lys Val Gly Asn Ala Asp Val Val Cys Gly Gly Val Ser
                   165                 170                 175
   Thr Ala Asn Ala Thr Val Tyr Met Ile Asp Ser Val Leu Met Pro Pro
               180                 185                 190
   Ala